# FASCICULE DE BREVET EUROPEEN

(11) **EP 0 784 454 B1**
(45) Date de publication et mention de la délivrance du brevet: **23.06.1999**
(21) Numéro de dépôt: 95940299.1
(22) Date de dépôt: 10.11.1995
(51) Int. Cl.: A61B 17/80

(54) **DISPOSITIF DE BLOCAGE TEMPORAIRE DE DEUX PARTIES D'UNE PIECE OSSEUSE**
EINRICHTUNG ZUR VORLÄUFIGEN FIXIERUNG ZWEIER KNOCHENTEILE
DEVICE FOR TEMPORARILY LOCKING TWO PORTIONS OF A BONE

(30) Priorité: 10.11.1994 FR 9413570
(43) Date de publication de la demande: 23.07.1997
(73) Titulaire: Kehyayan, Georges, 92140 Clamart (FR); Dufour, Guillaume, 92600 Asnières-sur-Seine (FR)
(72) Inventeur: Kehyayan, Georges, 92140 Clamart (FR); Dufour, Guillaume, 92600 Asnières-sur-Seine (FR)
(74) Mandataire: Puiroux, Guy
(86) Numéro de dépôt international: FR9501483
(87) Numéro de publication internationale: WO9614802

(56) Documents cités:
- WO-A-81/00048
- DE-B- 2 213 283
- FR-A- 2 676 353

## Description

La présente invention concerne un dispositif de blocage temporaire du type utilisé dans les interventions d'ostéotomie et notamment d'ostéotomie tibiale ouverte.

On sait que, dans ce type d'intervention, le chirurgien, après avoir réalisé une découpe transversale partielle de la partie supérieure d'une pièce osseuse et notamment d'un tibia, fait pivoter l'une par rapport à l'autre les deux parties découpées afin de redonner à l'angle formé par les axes anatomiques et mécaniques de ladite pièce osseuse la valeur souhaitée. Le maintien en position des deux parties découpées est ensuite habituellement assuré par des agrafes ou des plaques vissées qui sont fixées longitudinalement sur celles-ci.

On sait qu'une difficulté de ce type d'opération est d'assurer le maintien l'une par rapport à l'autre des deux parties découpées, à un écartement précis donné, pendant le temps nécessaire à la consolidation de l'os.

La présente invention a pour but de remédier à cet inconvénient en proposant un dispositif de blocage de deux parties découpées d'une pièce osseuse, et notamment d'un tibia, qui autorise un préréglage de l'écartement à prévoir entre ces deux parties pour leur donner l'angle souhaité, et qui assure un maintien positif à cet écartement donné jusqu'à la consolidation de l'os.

La présente invention a ainsi pour objet un dispositif de blocage temporaire de deux parties d'une pièce osseuse, séparées au cours d'une opération d'ostéotomie, comportant au moins un élément solidarisable des deux dites parties de la pièce osseuse, caractérisé en ce que cet élément comporte des moyens de butée, s'étendant transversalement à partir de sa face interne, dont l'écartement des faces extrêmes est égal à celui dont on souhaite écarter lesdites parties de la pièce osseuse et qui sont destinés à venir en appui contre celles-ci, de façon à assurer leur maintien avec ledit écartement déterminé.

Dans un premier mode de mise en oeuvre de l'invention les deux moyens de butée sont disposés sur une pièce unique, à une distance fixe prédéterminée, si bien que le chirurgien aura à sa disposition un échantillonnage de tels dispositifs dont les moyens de butée seront plus ou moins écartés en fonction du travail à effectuer.

Dans un second mode de mise en oeuvre intéressant de l'invention l'écartement des moyens de butée est réglable et le dispositif de blocage comporte ainsi deux éléments mobiles montés à déplacement longitudinal l'un par rapport à l'autre et respectivement solidarisables des deux parties de la pièce osseuse, et des moyens de blocage des deux éléments mobiles l'un par rapport à l'autre en plusieurs positions prédéterminées.

La mise en oeuvre d'un tel dispositif suivant l'invention est particulièrement aisée puisqu'il rend possible, avant sa mise en place, le préréglage de l'écartement des moyens de butées, et donc celui des deux faces de coupe des deux parties de la pièce osseuse, à la valeur souhaitée. Il est ainsi possible, avec un seul dispositif, d'obtenir une fourchette étendue d'écarts angulaires. De plus, il est facile d'obtenir une précision élevée car le préréglage peut être effectué en dehors des diverses contraintes techniques que l'on rencontre habituellement sur un site opératoire.

Dans une variante de ce mode de mise en oeuvre de l'invention, les deux éléments mobiles sont respectivement constitués d'une glissière, comportant un évidement longitudinal, et d'un coulisseau apte à se déplacer en différentes positions préréglables de celui-ci.

Les moyens de blocage peuvent être constitués notamment de dents, de formes complémentaires respectivement prévues sur les chants internes de l'évidement et sur les chants externes du coulisseau, ces dents étant sensiblement perpendiculaires à la face de contact du coulisseau et de la glissière avec la pièce osseuse.

On décrira ci-après, à titre d'exemples, différents mode de mise en oeuvre de l'invention en regard des dessins annexés sur lesquels:
La figure 1 est une vue en coupe longitudinale d'un dispositif de blocage suivant l'invention une fois mis en place sur un tibia.
La figure 2 est une vue en élévation du dispositif de blocage représenté sur la figure 1.
La figure 3 est une vue en perspective éclatée d'une autre forme de mise en oeuvre d'un dispositif de blocage suivant l'invention.
La figure 4 est une vue en coupe longitudinale du dispositif représenté sur la figure 3 une fois celui-ci mis en place sur un tibia.
La figure 5 est une vue en coupe partielle suivant la ligne V-V de la figure 4.
La figure 6 est une vue en perspective d'un troisième mode de mise en oeuvre d'un dispositif de blocage suivant l'invention.
La figure 7 est une vue en coupe du dispositif représenté sur la figure 6 suivant la ligne VII-VII de celle-ci.
La figure 8 est une vue en coupe longitudinale du dispositif représenté sur la figure 6 une fois celui-ci mis en place sur un tibia.
La figure 9 est une vue en coupe du dispositif représenté sur les figures 6 à 8, ce dispositif étant réglé dans une position d'écart minimal.
La figure 10 est une vue en plan partielle d'une variante de mise en oeuvre de l'invention.
La figure 11 est une vue en coupe partielle à plus grande échelle d'une variante de mise en oeuvre de l'invention.
La figure 12 est une vue en perspective d'une autre variante de mise en oeuvre de l'invention.

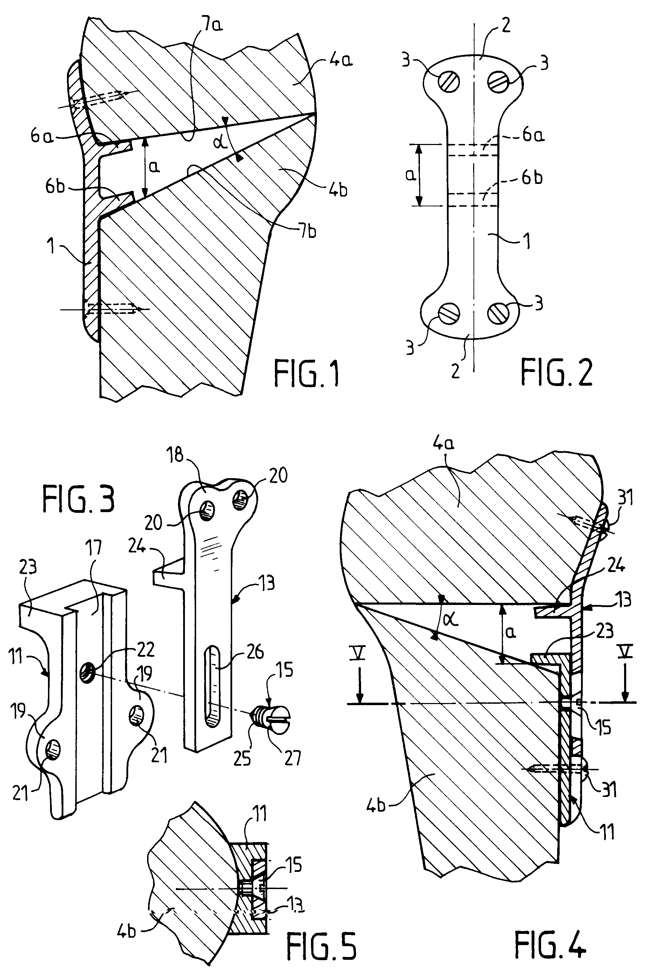

Le dispositif de blocage temporaire suivant l'invention représenté sur les figures 1 et 2 est constitué d'une plaquette 1 réalisée en un matériau biocompatible, et notamment en acier inoxydable, qui comporte à chacune de ses extrémités une tête 2 comportant chacune deux trous de fixation 3. La plaquette 1 comporte, sur sa face interne, c'est-à-dire sur la face destinée à entrer en contact avec chacune de deux parties 4a et 4b d'un tibia, deux pattes de maintien, à savoir une patte supérieure 6a et une patte inférieure 6b. Les pattes 6a et 6b ont leurs faces supérieure et inférieure extrêmes éloignées d'une distance a correspondant à celle dont on souhaite écarter les parties 4a et 4b du tibia, de façon que les faces 7a et 7b correspondant à la découpe effectuée forment un angle α de valeur souhaitée par le praticien.

De préférence, comme représenté sur la figure 1, les faces extrêmes externes des pattes 6a et 6b sont inclinées par rapport à la face interne de la plaquette 1 afin qu'elles soient sensiblement coplanaires aux faces 7a, 7b de façon à améliorer leur contact avec celles-ci.

Le praticien disposera d'un échantillonnage de plaquettes 1, dont l'écartement a des faces extrêmes des pattes 6a et 6b seront variées afin de lui permettre d'obtenir une large gamme d'angles α souhaités.

La mise en place de la plaquette 1 se fait de façon aisée puisque, une fois les pattes 6a, 6b glissées entre les deux parties 7a et 7b du tibia, ces dernières, en raison de la pression qu'elles exercent sur les pattes, assurent un maintien provisoire de la plaquette 1 pendant le temps nécessaire au chirurgien pour disposer dans les trous 3 des vis destinées à fixer la plaquette 1 sur les deux parties 4a et 4b du tibia.

Suivant la présente invention le chirurgien obtient facilement et avec une grande précision l'angle α souhaité, sans que les différentes contraintes qui se manifestent au cours d'une opération ne portent atteinte à la précision du travail effectué. Par ailleurs, les deux parties 4a et 4b de la pièce osseuse se trouvent complètement bloquées, ce qui favorise la reformation et la consolidation de l'os.

Bien entendu, la plaquette 1 peut être réalisée en deux parties, de façon à rendre réglable l'écartement a des faces extrêmes des pattes 6a et 6b.

Ainsi, le dispositif de blocage temporaire représenté sur les figures 3 à 5 comprend essentiellement trois éléments, à savoir une glissière 11, un coulisseau 13 et une vis de fixation 15.

La glissière 11 est constituée d'un élément allongé qui comporte une rainure axiale et longitudinale 17, destinée à recevoir le coulisseau 13, et deux oreilles latérales de fixation 19 pourvues de trous 21. La glissière 11 comporte en outre, à sa partie supérieure sur le dessin, une butée 23 qui s'étend transversalement du côté opposé à la rainure 17. Le fond de celle-ci est percé d'un trou fileté 22.

Le coulisseau 13 est constitué d'une plaque allongée dont les dimensions lui permettent de coulisser librement dans la rainure 17. L'une de ses extrémités, l'extrémité supérieure sur le dessin, comporte une double oreille de fixation 18 pourvue de deux trous 20. A proximité de l'oreille de fixation 18 le coulisseau 13 comprend une butée 24 qui s'étend dans la même direction que la butée 23 lorsque le coulisseau 13 est en place dans la rainure 17. Le coulisseau 13 comporte en outre une lumière longitudinale 26 de section trapézoïdale, la petite base du trapèze étant située du côté venant en contact avec le fond de la rainure 17.

La vis de fixation 15 est constituée d'une partie filetée 25, destinée à se visser dans le trou fileté 22 de la glissière 11, suivie d'une tête tronconique 27 dont la conicité est la même que celle de la lumière 26.

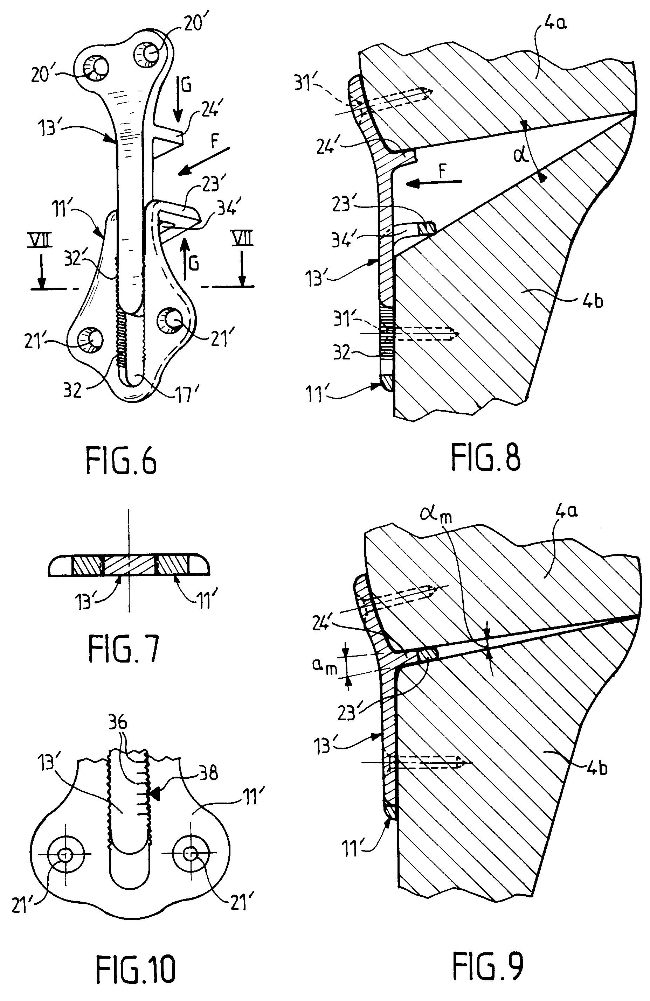

Dans ces conditions, comme représenté sur les figures 4 et 5, lorsque l'on souhaite immobiliser les deux parties 4a et 4b d'un tibia, de façon que ces deux parties forment entre elles un angle α souhaité, on positionne le coulisseau 13 dans la glissière 11 de façon que l'écartement a séparant les parties extrêmes des deux butées 23 et 24 ait une valeur appropriée, déterminée par le calcul, apte à produire ledit angle α, puis on immobilise le coulisseau 13 sur la glissière 11 en appliquant les flancs coniques de la vis 15 contre les bords de la lumière 26, ce que l'on obtient en serrant la vis 15. On présente ensuite le dispositif ainsi bloqué devant les deux éléments de tibia 4a et 4b et on introduit les deux butées 23 et 24 entre ces derniers. L'effort exercé par les deux éléments de tibia 4a et 4b, qui ont tendance à se rapprocher, assure le maintien du dispositif pendant le temps nécessaire à sa fixation, c'est à dire à la mise en place de vis de fixation 31 respectivement dans les éléments de tibia 4a et 4b, au travers des trous 20 et 21.

On a représenté sur les figures 6 à 9 une variante de mise en oeuvre particulièrement intéressante du dispositif de blocage suivant l'invention. Ce dispositif de blocage comprend une glissière 11' et un coulisseau 13'.

La glissière 11' est formée d'une plaque en acier inoxydable qui est traversée longitudinalement par une lumière 17' qui débouche à son extrémité supérieure. Le chant de la lumière 17' comporte, sur sensiblement toute sa longueur, des dents transversales 32 perpendiculaires au plan de la plaque. La partie supérieure de la glissière 11' est repliée, à environ 90°, de façon à former une butée 23'. La glissière 11' comporte des trous de fixation 21'.

Le coulisseau 13' est constitué d'un élément allongé en acier inoxydable dont la section droite a une forme complémentaire de celle de la lumière 17' et son chant comporte des dents transversales 32' de forme complémentaire des dents 32 de façon qu'il puisse prendre place dans la lumière 17' en une position quasiment quelconque de celle-ci et s'y trouver bloqué dans le sens longitudinal en raison de la coopération de ses dents 32' avec les dents 32 de la glissière 11'. Il comporte une butée 24' qui s'étend transversalement du même côté que la butée 23' lorsque le coulisseau 13' est en place dans la glissière 11'.

Le présent dispositif est particulièrement intéressant en ce qu'il assure un blocage longitudinal de la glissière 11' et du coulisseau 13' qui est totalement exempt de glissement tout en étant facile à régler. Par ailleurs, sa mise en place entre les deux parties de tibia 4a et 4b est aisée à mettre en oeuvre car, en exerçant un effort de compression sur les deux butées 23' et 24' suivant la direction des flèches G, on assure le maintien du coulisseau 13' dans la glissière 11', effort que le praticien pourra exercer sur les deux butées 23' et 24' en les saisissant par exemple entre le pouce et l'index pour les insérer entre les deux parties de tibia 4a, 4b. Une fois en place l'effort exercé par ces dernières sur les butées 23' et 24' continuera à assurer un tel maintien pendant le temps nécessaire à leur fixation avec des vis 31'. Une fois cette fixation réalisée, les deux parties 4a et 4b de la pièce osseuse se trouveront complètement bloquées pendant tout le temps nécessaire à la consolidation de l'os.

Le maintien de la glissière 13' dans le coulisseau 11' est grandement amélioré, notamment avant la mise en place de l'ensemble entre les deux éléments osseux à bloquer, en donnant à la lumière 17' et à la branche de la glissière 13' une section trapézoïdale, la petite base du trapèze étant située du côté de la pièce osseuse.

Dans une variante de ce mode de mise en oeuvre de l'invention, représentée sur les figures 8 et 9, l'une des butées, à savoir la butée 23' dans le présent exemple, est creusée d'une cavité 34' dont les dimensions sont légèrement supérieures à celles de l'autre butée 24'. Il est ainsi possible à la butée 24', ainsi que représenté sur la figure 9, de venir se loger dans la cavité 34', ce qui permet de réaliser un écart minimal am entre les faces externes des butées 23' et 24' qui est égal à une épaisseur de butée, et d'obtenir ainsi un angle αm minimal de faible valeur de l'ordre de deux degrés.

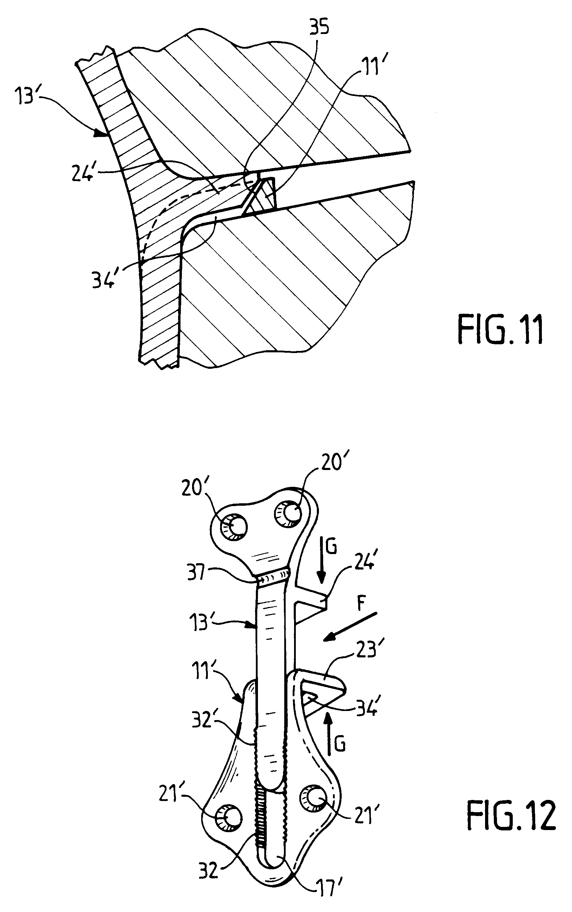

Afin d'augmenter la longueur de la portée de la butée 24' sur l'élément de pièce osseuse 4a on peut, comme représenté sur la figure 11, réaliser une cavité 34' dont au moins la paroi interne située du côté de la pièce osseuse, lorsque le dispositif de blocage est en place, comporte un chanfrein 35 incliné du haut vers le bas et de l'extérieur vers l'intérieur, et donner à la face externe de la butée 24' du coulisseau 13' une inclinaison complémentaire.

On peut également, ainsi que représenté sur la figure 12, prévoir, légèrement au-dessus de la butée 24', et d'un côté au moins du coulisseau 13', une zone de moindre résistance, notamment au moyen d'une rainure 37 de section droite de préférence arrondie. Cette rainure 37 a pour but de réduire, dans cette zone, l'épaisseur du coulisseau 13'. Ce mode de mise en oeuvre permet, lorsque les deux éléments 4a et 4b de la pièce osseuse à réunir ne forment pas l'angle exact présenté par la glissière 11' et le coulisseau 13', de plier ces derniers de façon qu'ils viennent en portée l'un et l'autre sur les éléments osseux 4a et 4b.

Dans une autre variante de ce mode de mise en oeuvre de l'invention, représentée sur la figure 10, on a gravé une série de graduations 36 sur le coulisseau 13' et un repère 38 sur la glissière 11', les graduations 36 pouvant être marquées avec des valeurs indiquant, lorsqu'elles se trouvent en face du repère 38, l'écartement qui existe entre les butées 23' et 24'. On pourra également bien entendu, sur la base d'un diamètre moyen de tibia réaliser directement un tel marquage en degrés, correspondant à l'angle α souhaité.

Dans un autre mode de mise en oeuvre de l'invention, la glissière 11' est incurvée transversalement de façon à mieux épouser la forme de la pièce osseuse. On lui donne ainsi un certain rayon de courbure dans le sens transversal voisin du rayon de courbure moyen de la pièce osseuse.

On peut améliorer le contact du coulisseau 13' avec la pièce osseuse en lui donnant une épaisseur telle que, lorsqu'il est en place dans la glissière 11', il dépasse légèrement de celle-ci du côté destiné à entrer en contact avec la pièce osseuse. Cette disposition permet au coulisseau 13' de venir en contact avec la pièce osseuse, même lorsque le rayon de courbure dans le sens transversal de la pièce osseuse est supérieur à celui de la glissière 11'.

Bien que les dispositifs de blocage aient été précédemment décrits dans des applications d'ostéotomie tibiale il est bien entendu qu'ils pourraient être également utilisés dans des interventions réalisées sur d'autres pièces osseuses.

## Revendications

1. Dispositif de blocage temporaire de deux parties (4a,4b) d'une pièce osseuse séparées au cours d'une opération d'ostéotomie, comportant au moins un élément (1,11,11',13,13') solidarisable des deux dites parties (4a,4b) de la pièce osseuse, caractérisé en ce que cet élément comporte des moyens de butée (6a,6b,23,23',24,24'), s'étendant transversalement à partir de sa face interne, dont l'écartement (a) des faces externes est égal à celui dont on souhaite écarter lesdites parties (4a,4b) de la pièce osseuse et qui sont destinés à venir en appui contre celles-ci, de façon à assurer leur maintien avec ledit écartement (a) détermine.

2. Dispositif suivant la revendication 1 caractérisé en ce que ledit élément comporte une zone de moindre résistance (37) destinée à favoriser sa courbure.

3. Dispositif suivant l'une des revendications 1 ou 2, caractérisé en ce qu'il comporte deux éléments mobiles (11,11',13,13') montés à déplacement longitudinal l'un par rapport à l'autre et respectivement solidarisables des deux parties (4a,4b) de ladite pièce osseuse, et des moyens de blocage (15,32) des deux éléments mobiles l'un par rapport à l'autre en plusieurs positions prédéterminées.

4. Dispositif suivant la revendication 3 caractérisé en ce que les deux éléments mobiles sont respectivement constitués d'une glissière (11,11') comportant un évidemment longitudinal (17,17') et d'un coulisseau (13,13'), apte à se déplacer en différentes positions préréglables de celui-ci.

5. Dispositif suivant la revendication 4 caractérisé en ce que l'évidement longitudinal (17,17') et le coulisseau (13,13') ont une section trapézoïdale, la petite base du trapèze étant située du côté destiné à être appliqué contre la pièce osseuse.

6. Dispositif suivant l'une des revendications 3 à 5 caractérisé en ce que les moyens de blocage sont constitués de dents (32), de formes complémentaires respectivement prévues sur les chants internes de l'évidement (17') et sur les chants externes du coulisseau (13'), ces dents (32) étant sensiblement perpendiculaires à la face de contact du coulisseau (13') et de la glissière (11') avec la pièce osseuse.

7. Dispositif suivant l'une des revendications 3 à 6 caractérisé en ce qu'il comporte une première butée (23') solidaire d'un élément mobile (11') qui est pourvue d'une cavité (34') apte à recevoir, au moins en partie, une seconde butée (24') solidaire de l'autre élément mobile (13').

8. Dispositif suivant la revendication 7 caractérisé en ce que au moins la paroi interne de la cavité (34') située du côté de la pièce osseuse, lorsque le dispositif de blocage est en place, comporte un chanfrein (35) incliné du haut vers le bas et de l'extérieur vers l'intérieur, et la face externe correspondante de la seconde butée (24') possède une inclinaison complémentaire.

9. Dispositif suivant l'une des revendications 3 à 8 caractérisé en ce que l'un des éléments mobiles (11,11',13,13') comporte des graduations (36), l'autre élément mobile étant pourvu d'un repère (38) permettant de connaître l'écartement (a) des éléments de butée (23,23',24,24') et/ou l'angle (α) formé par les deux parties (4a,4b) de la pièce osseuse, sur la base d'un diamètre moyen de tibia lorsque le dispositif est en place sur la pièce osseuse.

10. Dispositif suivant l'une des revendications précédentes caractérisé en ce que ledit élément (1,11,11',13,13') solidarisable des deux parties (4a,4b) de la pièce osseuse comporte au moins une partie incurvée dans le sens transversal.

## Claims

1. Device for temporarily locking two portions (4a, 4b) of a bone separated during an osteotomy operation, comprising at least one element (1, 11, 11', 13, 13') adapted to be connected to the said two portions (4a, 4b) of the bone, characterized in that this element comprises abutment means (6a, 6b, 23, 23', 24, 24'), extending tranversely from its inner face, of which the spacing (a) of the outer faces is equal to that by which it is desired to separate said portions (4a, 4b) of the bone and which are intended to come into abutment thereagainst, so as to ensure hold thereof with said determined spacing (a).

2. Device according to Claim 1, characterized in that said element comprises a zone of least resistance (37) intended to promote curvature thereof.

3. Device according to one of Claims 1 or 2, characterized in that it comprises two mobile elements (11, 11', 13, 13') mounted for longitudinal displacement with respect to each other and adapted to be respectively connected with the two portions (4a, 4b) of said bone, and means (15, 32) for locking the two elements mobile with respect to each other in a plurality of predetermined positions.

4. Device according to Claim 3, characterized in that the two mobile elements are respectively constituted by a slideway (11, 11') comprising a longitudinal recess (17, 17') and by a slide block (13, 13') adapted to move in different pre-adjustable positions of said block.

5. Device according to Claim 4, characterized in that the longitudinal recess (17, 17') and the slide block (13, 13') present a trapezoidal section, the small base of the trapezium being located on the side intended to be applied against the bone.

6. Device according to one of Claims 3 to 5, characterized in that the locking means are constituted by teeth (32), of complementary shapes respectively provided on the inner edges of the recess (17') and on the outer edges of the slide block (13'), these teeth (32) being substantially perpendicular to the face of contact of the slide block (13') and of the slideway (11') with the bone.

7. Device according to one of Claims 3 to 6, characterized in that it comprises a first abutment (23') fast with a mobile element (11') which is provided with a cavity (34') adapted to receive, at least partially, a second abutment (24') fast with the other mobile element (13').

8. Device according to Claim 7, characterized in that at least the inner wall of the cavity (34') located towards the bone, when the locking device is in place, comprises a bevel (35) inclined downwardly and inwardly, and the corresponding outer face of the second abutment (24') presents a complementary inclination.

9. Device according to one of Claims 3 to 8, characterized in that one of the mobile elements (11, 11', 13, 13') comprises graduations (36), the other mobile element being provided with a mark (38) making it possible to know the spacing (a) of the abutment elements (23, 23', 24, 24') and/or the angle (α) formed by the two portions (4a, 4b) of the bone, on the basis of a mean tibia diameter when the device is in place on the bone.

10. Device according to one of the preceding Claims, characterized in that said element (1, 11, 11', 13, 13') adapted to be connected to the two portions (4a, 4b) of the bone comprises at least one part incurved in the transverse direction.

## Patentansprüche

1. Vorrichtung zum temporären Festklemmen zweier, während einer Osteotomie- bzw. Knochendurchtrennungsoperation getrennter Abschnitte (4a, 4b) eines Knochenstücks, welche Vorrichtung mindestens ein Element (1, 11, 11', 13, 13') aufweist, welches mit den beiden Abschnitten (4a, 4b) des Knochenstücks fest verbindbar ist,
dadurch gekennzeichnet, daß dieses Element Anschlagmittel (6a, 6b, 23, 23', 24, 24') aufweist, die sich transversal ausgehend von seiner Innenfläche erstrecken, deren Abstand (a) von den Außenseiten gleich demjenigen ist, in welchem die Abschnitte (4a, 4b) des Knochenstücks gespreizt werden sollen, und die dazu bestimmt sind, gegen die letztgenannten Abschnitte in Anlage zu kommen, so daß ihr Halt bei dem bestimmten Abstand (a) gewährleistet ist.

2. Vorrichtung nach Anspruch 1,
dadurch gekennzeichnet, daß das Element eine Zone (37) geringen Widerstands aufweist, die dazu bestimmt ist, seine Biegung zu fördern.

3. Vorrichtung nach Anspruch 1 oder 2,
dadurch gekennzeichnet, daß sie zwei bewegliche Elemente (11, 11', 13, 13'), die in Längsverschiebung zueinander angebracht und jeweils mit den beiden Abschnitten (4a, 4b) des Knochenstücks fest verbindbar sind, und Mittel (15, 32) zum Festklemmen der beiden zueinander beweglichen Elemente in mehreren vorbestimmten Positionen aufweist.

4. Vorrichtung nach Anspruch 3,
dadurch gekennzeichnet, daß die beiden beweglichen Elemente jeweils aus einer Gleitschiene (11, 11'), die eine längliche Aussparung (17, 17') aufweist, und einem Schlitten (13, 13') gebildet sind, der in verschiedenen vorregelbaren Position von dieser letztgenannten verschoben werden kann.

5. Vorrichtung nach Anspruch 4,
dadurch gekennzeichnet, daß die längliche Aussparung (17, 17') und der Schlitten (13, 13') einen trapezförmigen Querschnitt haben, wobei die kleine Basis des Trapezes auf der Seite liegt, die dazu bestimmt ist, an das Knochenstück aufgedrückt zu werden.

6. Vorrichtung nach einem der Ansprüche 3 bis 5,
dadurch gekennzeichnet, daß die Festklemm-Mittel aus Zähnen (32) komplementärer Formen gebildet sind, welche Zähne jeweils an den Innenkanten der Aussparung (17') und an den Außenkanten des Schlittens (13') vorgesehen sind, wobei diese Zähne (32) im wesentlichen senkrecht zur Kontaktfläche des Schlittens (13') und der Gleitschiene (11') mit dem Knochenstück sind.

7. Vorrichtung nach einem der Ansprüche 3 bis 6,
dadurch gekennzeichnet, daß sie einen ersten Anschlag (23') aufweist, der mit einem beweglichen Element (11') fest verbunden ist, welcher Anschlag mit einer Vertiefung (34') versehen ist, die mindestens teilweise einen zweiten Anschlag (24') aufnehmen kann, der mit dem anderen beweglichen Element (13') fest verbunden ist.

8. Vorrichtung nach Anspruch 7,
dadurch gekennzeichnet, daß mindestens die Innenwand der Vertiefung (34'), die auf der Seite des Knochenstücks liegt, wenn die Festklemmvorrichtung in Stellung ist, eine Fase (35) aufweist, die von oben nach unten und von außen nach innen geneigt ist, und die entsprechende Außenfläche des zweiten Anschlags (24') besitzt eine komplementäre Neigung.

9. Vorrichtung nach einem der Ansprüche 3 bis 8,
dadurch gekennzeichnet, daß eines der beweglichen Elemente (11, 11', 13, 13') Graduierungen (36) aufweist, wobei das andere bewegliche Element mit einer Markierung (38) versehen ist, die gestattet, den Abstand (a) der Anschlagelemente (23, 23', 24, 24') und/oder den Winkel (α) zu kennen, der durch die beiden Abschnitte (4a, 4b) des Knochenstücks an der Basis eines mittleren Durchmessers einer Tibia bzw. eines Schienbeins gebildet ist, wenn die Vorrichtung an dem Knochenstück in Stellung ist.

10. Vorrichtung nach einem der vorhergehenden Ansprüche,
dadurch gekennzeichnet, daß das Element (1, 11, 11', 13, 13'), welches mit den beiden Abschnitten (4a, 4b) des Knochenstücks fest verbindbar ist, mindestens einen in der transversalen Richtung gebogenen Abschnitt aufweist.
